# EUROPÄISCHE PATENTSCHRIFT

(11) **EP 1 166 104 B1**
(45) Veröffentlichungstag und Bekanntmachung des Hinweises auf die Patenterteilung: **09.07.2003**
(21) Anmeldenummer: 00906261.3
(22) Anmeldetag: 01.02.2000
(51) Int. Cl.: G01N 33/24, G01N 15/08

(54) **VORRICHTUNG ZUR UNTERSUCHUNG VON UMWELTCHEMIKALIEN IN BÖDEN MITTELS EINES DYNAMISCHEN LABOR-TESTSYSTEMS**
DEVICE FOR INVESTIGATING ENVIRONMENTAL CHEMICALS IN SOILS USING A DYNAMIC LABORATORY TESTING SYSTEM
DISPOSITIF PERMETTANT D'ETUDIER LES PRODUITS CHIMIQUES ENVIRONNEMENTAUX CONTENUS DANS LE SOL AU MOYEN D'UN SYSTEME DYNAMIQUE D'ANALYSES DE LABORATOIRE

(30) Priorität: 11.02.1999 DE 19905734
(43) Veröffentlichungstag der Anmeldung: 02.01.2002
(73) Patentinhaber: GSF-Forschungszentrum für Umwelt und Gesundheit GmbH, 85764 Oberschleissheim (DE)
(72) Erfinder: SCHROLL, Reiner, D-86551 Aichach (DE)
(74) Vertreter: Maiwald Patentanwalts GmbH
(86) Internationale Anmeldenummer: EP0000792
(87) Internationale Veröffentlichungsnummer: WO00047995

(56) Entgegenhaltungen:
- SU-A- 1 335 829
- US-A- 4 606 227
- US-A- 5 202 033
- US-A- 5 299 140

## Beschreibung

Die Erfindung betrifft eine Vorrichtung zur Untersuchung des Verhaltens und des Abbaus von Umweltchemikalien, insbesondere von Agrochemikalien, im dynamischen System, wobei die Vorrichtung mindestens ein Behältnis, gefüllt mit einem gestörten oder ungestört entnommenen Boden, einer oberhalb der Bodenoberfläche angeordnete Beregnungsvorrichtung, eine eine gleichmäßige Windbewegung oberhalb der Bodenoberfläche erzeugende Ventilatorvorrichtung und ein Unterdrucksystem zur Einstellung eines vorbestimmten Grundwasserspiegels innerhalb des Bodens umfaßt.

Das erfindungsgemäße Labortestsystem, im folgenden auch Mikrokosmosanlage genannt, ermöglicht die Bestimmung der Verlagerung, des Transports, der Umwandlung, der Mineralisierung und der Verflüchtigung von Chemikalien unter natürlichen Klimabedingungen sowohl ohne den Einfluß von Pflanzen als auch unter dem Einfluß von Pflanzen in ungestörten und gestörten Bodensäulen. Die Erfindung betrifft auch ein Verfahren zur Testung von Chemikalien in Böden unter Verwendung der erfindungsgemäßen Vorrichtung.

Im Gegensatz zu bekannten Testsystemen ermöglicht die erfindungsgemaße Mikrokosmosanlage die Untersuchung von Umweltchemikalien wie z.B. Pflanzenschutzmitteln, Düngemitteln u.ä., in einem dynamischen System, in dem natürliche Verhältnisse, insbesondere natürliche Klimaeinflüsse simuliert werden. Bekannte Systeme bieten diese Möglichkeit nicht. So erwähnen beispielsweise die von der Biologischen Bundesanstalt für Land- und Forstwirtschaft, Braunschweig, herausgegebenen "Richtlinien für die amtliche Prüfung von Pflanzenschutzmitteln" im Zusammenhang mit Metabolismusuntersuchungen im Labor meßbare Einzelparameter wie Verlagerung oder Mineralisierung, doch haben die laut den Richtlinien zu verwendenden Böden die wesentlichen Charakteristika von Böden im Feld (z.B. Bodenstruktur, Wassergehalt, usw.) verloren, da der Boden im Rahmen der Versuchsvorbereitung durch ein 2 mm-Sieb getrieben wird (siehe z.B. Seite 12 der Richtlinien, Teil IV, 4-1, Dezember 1986). Ferner wird bei den bisher üblichen Labortestverfahren durch die bei der Bodenvorbereitung durchgeführten Arbeitsschritte der Boden weiter verändert. So hat das Trocknen des Bodens z.B. zur Folge, daß ein großer Anteil von organischen Substanzen und von Nährstoffen aus abgetöteten Mikroorganismen verfügbar wird. Bei Wiederbefeuchten des Bodens kommt es so zu einer veränderten Mikroorganismengesellschaft mit veränderter Leistung. Weiter werden bei der Homogenisierung (z.B. durch das Sieben) des Bodens Energie- und Nährstoffquellen exponiert, die sonst den Mikroorganismen aufgrund räumlicher Strukturen bzw. Aggregierungen nicht zugänglich sind. Auch Sorptionsparameter werden im Stand der Technik in der Regel im Batchversuch oder an gepackten Säulen durchgeführt. Bei diesen Prozeduren wird jedoch die gesamte vorhandene adsorptionsfähige Oberfläche exponiert, d.h. man bestimmt die Adsorptionskapazität des Bodens, die in einem natürlich gelagerten Boden infolge von Makroporenflüssen (Wasserfluß durch Wurmgänge, leere Pflanzenwurzelgänge, Bodenrisse infolge von Bodenschrumpfungsprozessen) nicht immer wirksam ist.

US-A-5 299 140 offenbart eine Vorrichtung zur Untersuchung der physikalischen und chemischen Eigenschaften von porösen Proben, insbesondere einer Bodenprobe, die sich in einem geschlossen Behälter befindet. Dieser Behälter ist mit einem System verbunden, das sämtliche Bedingungen z.B. Luft- und Feuchtigkeitszufuhr, Druck und Temperatur kontrolliert.

Die vorliegende Erfindung soll insbesondere die Untersuchung von Agrochemikalien in sogenannten ungestört entnommenen Bodensäulen ermöglichen und dadurch die beschriebenen Nachteile des Standes der Technik vermeiden helfen. Es ist eine wesentliche Aufgabe der Erfindung, ein Labortestsystem zur Verfügung zu stellen, mit dem das Verhalten von Umweltchemikalien im System Wasser/Boden/Luft, gegebenenfalls im System Wasser/ Boden/Pflanze/Luft, untersucht werden kann. Hierbei soll es insbesondere möglich sein, die Verlagerung, die Umwandlung bis hin zur Mineralisierung, die Auswaschung und die Verflüchtigung von Umweltchemikalien sowohl ohne den Einfluß von Pflanzen, als auch unter dem Einfluß von Pflanzen in ungestörten oder in gestörten Bodensäulen zu untersuchen. In einer bevorzugten Ausführungsform handelt es sich um radioaktiv markierte, vorzugsweise ¹⁴C-markierte, Pflanzenschutzmittel bzw. andere Umweltchemikalien.

Diese und weitere Aufgaben, die sich aus der nachfolgenden Beschreibung ergeben, werden durch die vorliegende Erfindung gelöst, bei der es sich im Gegensatz zu allen bekannten Testsystemen um ein dynamisches Testsystem handelt, in dem natürliche Klimaeinflüsse, wie z.B. Niederschlagsereignisse, Grundwasserstände und Windbewegungen über der Bodenoberfläche simuliert werden.

Das erfindungsgemäße Labortestsystem umfaßt mindestens ein Behältnis, vorzugsweise ein zylinderförmiges Behältnis aus Metall, Glas, Teflon oder einem sonstigen inerten Material, das mit einer gestörten oder ungestörten Bodensäule gefüllt ist, eine oberhalb der Bodenoberfläche angeordnete Beregnungsvorrichtung, eine Ventilatorvorrichtung, die eine gleichmäßige Windbewegung oberhalb der Bodenoberfläche erzeugt, und ein Unterdrucksystem zur Einstellung des gewünschten Grundwasserspiegels innerhalb der Bodensäule. Mit Hilfe der Isotopentracertechnik, insbesondere der ¹⁴C-Markierung, in Kombination mit konventionellen analytischen Methoden ermöglicht das erfindungsgemäße System gezielte Untersuchungen zur Aufnahme von Pflanzenschutzmitteln durch Pflanzen sowie zum Langzeitverhalten von Rückständen im System Boden/Pflanze/Wasser/Luft. Besondere Berücksichtigung finden dabei. Detailprozesse wie Abbau und Metabolisierung, Sorption, Verlagerung im Boden, Bildung und Bioverfügbarkeit von gebundenen Rückständen sowie gasförmige Verluste durch Mineralisierung und Verflüchtigung. Des weiteren können sämtliche Rückstände der Umweltchemikalien, die von den Pflanzen über die Wurzeln aufgenommen und über die Blätter in die Atmosphäre abgegeben werden, kontinuierlich gemessen werden. Im Gegensatz zu allen bekannten Testsystemen ist die erfindungsgemäße Mikrokosmosanlage ein dynamisches Testsystem, in dem natürliche Klimaeinflüsse wie Niederschlagsereignisse, Grundwasserstände und Windbewegung über der Bodenoberfläche nachgestellt werden.

Durch den erfindungsgemäßen Versuchsaufbau als geschlossenes System ist die Aufstellung einer vollständigen Massenbilanz für die markierte Umweltchemikalie, die auf der Bodenoberfläche appliziert wird, möglich.

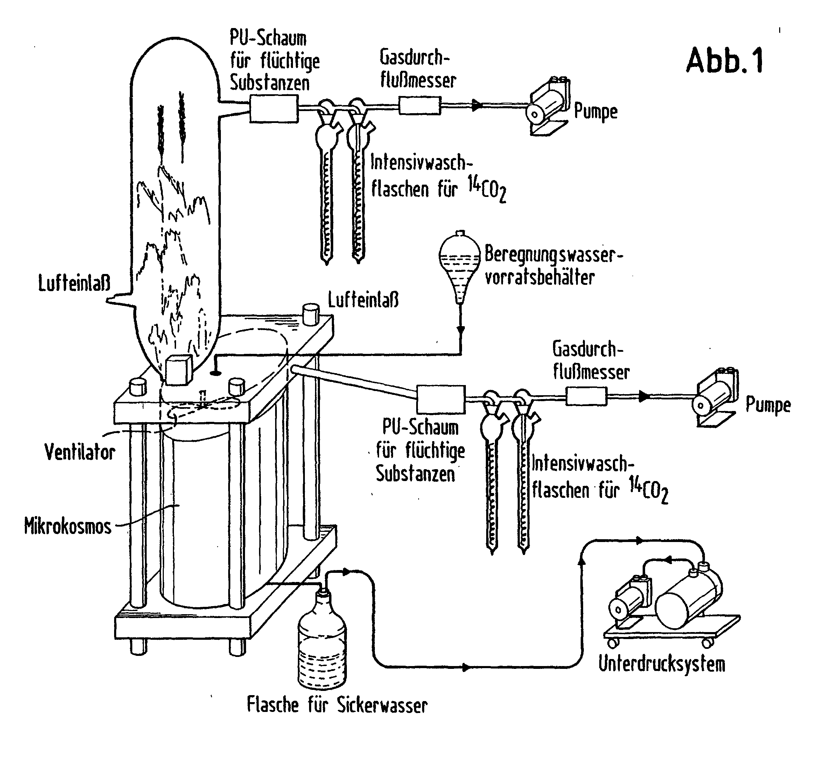

Die erfindungsgemäße Testanlage wird im folgenden unter Bezugnahme auf die beigefügte Abbildung 1 beschrieben, die eine bevorzugte Ausführungsform der Erfindung zeigt.

Das Zentrum der Labortestanlage bildet der Mikrokosmos, d.h. ein mit einer gestörten oder ungestört entnommenen Bodensäule gefülltes Behältnis. Vorzugsweise handelt es sich um ein zylinderförmiges Gefäß aus Metall, Glas, Teflon oder einem anderen geeigneten Material, welches inert ist, also die Massenbilanz nicht beeinflußt. Vorzugsweise hat das Behältnis einen Durchmesser von ca. 15 cm und eine Höhe von ca. 30 cm. Am unteren Ende ist das Behältnis mit einer Lochplatte und einem Filterpapier nach unten hin abgeschlossen, wobei der Filter vorzugsweise eine Porengröße im Bereich von 0,45 im aufweist. Vorzugsweise wird ein Polyamidfilter verwendet, obgleich auch andere Materialien als Filter eingesetzt werden können, solange eine übermäßige Sorption der zu testenden Substanz nicht stattfindet.

Am oberen Ende des mit Boden gefüllten Mikrokosmos ist ein besonders gestalteter Deckel angebracht, in welchen eine Beregnungseinheit eingearbeitet ist. Diese Beregnungsvorrichtung ist mit einem außerhalb liegenden Beregnungswasservorratsbehälter verbunden. Vorzugsweise wird natürliches Regenwasser oder eine 0,01 M CaCl₂-Lösung zur Beregnung des Bodens verwendet. Destilliertes Wasser ist wegen der im Boden enthaltenen mehrwertigen Kationen weniger als Beregnungsflüssigkeit geeignet.

Der den Mikrokosmos nach oben hin abschließende Deckel ist des weiteren derart konstruiert, daß vorgefilterte Luft über die Bodenoberfläche gesogen werden kann. Dabei kann das Kohlendioxid beispielsweise mit Hilfe von Natronkalk-Pellets aus der Luft gefiltert werden.

Eine Ventilatorvorrichtung, die mittig in dem Deckel angeordnet ist, verwirbelt die Luft über der Bodenoberfläche und sorgt so für eine gleichmäßige Windbewegung und -geschwindigkeit über der Bodenoberfläche, wobei der Ventilator je nach gewählter Anordnung der Beregnungsvorrichtung während des Beregnungsvorganges gestoppt werden muß. Der Ventilator bzw. eine Ventilatorähnliche Vorrichtung kann durch Luftzufuhr über eine Druckluftleitung, durch einen Elektromotor oder eine andere Antriebsquelle angetrieben werden, wobei die Antriebsquelle die Justierung der Windbewegungsgeschwindigkeit ermöglichen sollte.

In der Natur, d.h. bei Feldapplikation von Pflanzenschutzmitteln, diffundiert das Mittel nach der Bodenoberflächenapplikation zunächst vom Boden in eine dünne Grenzschicht (sogenannter boundary layer) oberhalb der Bodenoberfläche und nachfolgend in darüber liegende Luftschichten. Diese Verhältnisse werden durch die simulierte Windbewegung oberhalb der Bodenoberfläche im Mikrokosmos nachgestellt. Durch diese Luftbewegungen über der Bodenoberfläche werden Volatilitätstests unter quasi-natürlichen Bedingungen ermöglicht. Dabei wird die Luft anschließend durch einen Polyurethanschaumfilter und eine odere mehrere, vorzugsweise mindestens zwei, sogenannte Intensivwaschflaschen gesaugt. Anstelle des PU-Schaums kann jeder andere für volatile Substanzen geeignete Filter, beispielsweise eine Flüssigkeitsfalle, eingesetzt werden.

Die Intensivwaschflaschen sind mit einer reaktiven Flüssigkeit, vorzugsweise mit einer Mischung aus Ethanolamin und Diethylenglycolmonobutylether (z.B im Verhältnis 3:8,5) gefüllt. Durch ein Glasrohr wird die zu filternde Luft an das untere Ende der Intensivwaschflasche gesaugt und durch eine Glaswendel zwangsweise wieder nach oben geführt. Durch die Form der Glasteile und den durch eine Pumpe erzwungenen Luftstrom entstehen kleine Luftblasen, die entlang der Glaswendel nach oben perlen. Dadurch wird die Luft intensiv "ausgewaschen". Derartige "Fallensysteme" sind beispielsweise beschrieben in Schroll R. and Scheunert I. (1992) Chemosphere, Vol. 24, No. 1, Seiten 97-108. In einer bevorzugten Ausführungsform befindet sich am oberen Ende der Intensivwaschflaschen eine "Kühlzone", die mit Hilfe eines Kryostaten gekühlt wird und an der Flüssigkeitsmoleküle, die mit dem Luftstrom mitgerissen werden, kondensieren und wieder in die eigentliche Intensivwaschflasche zurücktropfen können.

Mit Hilfe eines Gasdurchflußmessers oder eines ggf. PC-gesteuerten "Mass-flowcontrollers" (MFC) wird der Luftstrom durch das gesamte Testsystem auf ein vorbestimmtes Maß (vorzugsweise im Bereich von etwa 23 l/h) eingestellt. Eine Membranpumpe oder Drehschieberpumpe hinter dem Gasdurchflußmesser bzw. MFC sorgt für den entsprechenden Luftfluß durch das System.

Das untere Ende der Mikrokosmosanlage ist über einen Teflonschlauch mit einer Glasflasche und einer Membranpumpe mit einem hydraulischen Unterdruckventil verbunden. Anstelle von Teflonschläuchen können auch andere Schläuche aus inertem Material eingesetzt werden, wobei Kunststoff weniger geeignet ist, da bei derartigen Materialien eine unerwünschte Beeinflussung der Massenbilanz nicht ausgeschlossen werden kann.

Die Glasflasche, in der das Sickerwasser gesammelt wird, ist vorzugsweise in einem Kühlschrank (etwa 4 °C) positioniert. Durch das Unterdrucksystem kann der Grundwasserspiegel innerhalb der Bodensäule eingestellt werden. Vorzugsweise wird ein Unterdruck von maximal 200 mbar eingestellt, um einen Grundwasserspiegel zu erreichen, der den Verhältnissen im Feld möglichst nahe kommt.

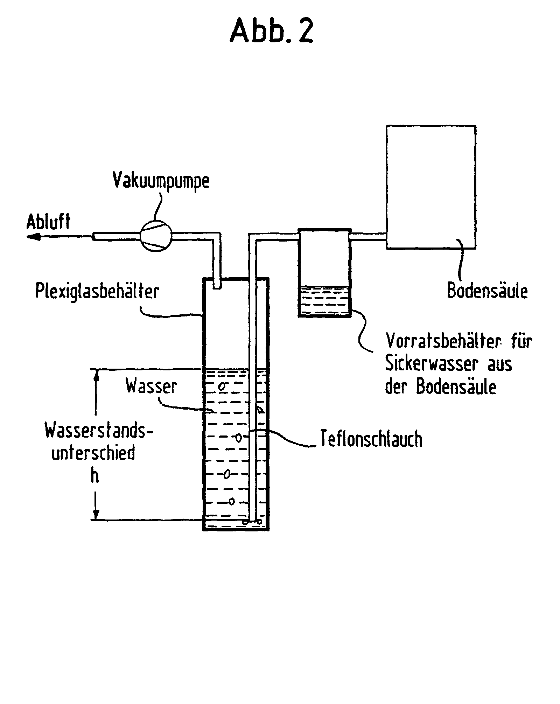

Die Einstellung des Unterdrucks kann beispielsweise mittels eines hydraulischen Sicherheitsventils erfolgen. Ein geeignetes Unterdrucksystem ist in Abbildung 2 veranschaulicht. Ist der erreichte Endunterdruck (gegen Atmosphärendruck) der Vakuumpumpe größer als erwünscht, so stellt das hydraulische Druckventil in der Bodensäule einen einstellbaren und konstanten Unterdruck von p = ñ·g·h sicher. Dieser Unterdruck kann durch das Ausmaß der Befüllung des in Abbildung 2 gezeigten Behälters mit Wasser eingestellt werden. Bei steigendem Unterdruck im Behälter sinkt der Wasserstand im Teflonschlauch bzw. steigt der Unterdruck in der Bodensäule, gleichzeitig steigt der Wasserstand im Behälter. Der Wasserstandsunterschied h nimmt zu. Ist das Wasser bis zum untersten Ende des Teflonschlauchs aus diesem verdrängt, so nimmt h den Maximalwert ein. Erhöht sich der Unterdruck im Behälter noch weiter, so treten am Teflonschlauch Luftbläschen aus. Der Wasserstandsunterschied sowie der Unterdruck in der Bodensäule bleibt konstant. Soll z.B. der Unterdruck in der Bodensäule auf 30 mbar eingestellt werden, muß der Wasserstandsunterschied (Behälter zu Teflonschlauch) bei Pumpbetrieb und aufsteigenden Gasbläschen 30 cm betragen (30 hPa = 1000 kg/m³ x 10 m/s² x h => h = 0,3 m).

Etwas unterhalb der oberen Kante des Mikrokosmosgefäßes ist ein ca. 4 cm langes Rohr mit einem Durchmesser von ca. 2 cm seitlich in einem Winkel von 45° angebracht. Dieses Rohr kann mit einer Irisblende nahezu vollständig verschlossen werden. Dem gegenüber ist an einer speziellen Halterung ein unten geschlossenes Glasrohr (Durchmesser ca. 10 cm, Höhe ca. 20 cm) angebracht, an dem ebenfalls in einem Winkel von 45° ein Glasrohr (Durchmesser ca. 2 cm) mit einer Irisblende angebracht ist. Am unteren Ende des Glasrohres befindet sich eine Glasolive als Lufteinlaßöffnung und eine zweite Öffnung mit einem Teflonhahn, um eventuell auftretendes Kondenswasser ablassen zu können. Das große Glasrohr wird nach oben durch eine zweites Glasrohr (Durchmesser ca. 10 cm, Höhe ca. 80 cm) abgeschlossen. Am oberen Ende des oberen Glasrohres befindet sich eine weitere Schlaucholive. Über einen Teflonschlauch ist diese Schlaucholive mit einem zweiten "Fallensystem" verbunden, d.h. wie oben beschrieben wird die Luft aus dem Glasrohr mit Hilfe einer zweiten Membranpumpe durch einen Polyurethanschaumfilter und zwei Intensivwaschflaschen gesaugt. Mit Hilfe eines Gasdurchflußmessers bzw. MFC wird der Luftstrom durch das gesamte Testsystem (wie oben erwähnt vorzugsweise auf etwa 23 l/h) eingestellt.

Wenn der Einfluß von Pflanzen auf das Verhalten von Chemikalien in Böden untersucht werden soll, dann sollten die Pflanzen vor dem eigentlichen Versuch vorgekeimt werden. Damit die Pflanzen im System gut gehandhabt werden können, sollten die oberirdischen Pflanzenteile eine gewisse Mindestwuchshöhe erreicht haben. Diese liegt bevorzugt bei etwa 7 - 15 cm. Für die Anzucht der Testpflanzen, beispielsweise Gerste, Weizen, Hafer, Mais oder Raps, werden kleine Kunststoffgefäße mit Boden gefüllt und pro Gefäß ein Samenkorn etwa 1 - 2 cm tief in den Boden gedrückt und mit Wasser angegossen. In den nachfolgenden Tagen wird die Bodenfeuchte kontrolliert und bei Bedarf gegossen.

Soll ein ungestört entnommener Boden als Versuchsmaterial verwendet werden, so wird vorzugsweise der oben beschriebene Zylinder zum Ausstechen der Säule verwendet. Bevor die Bodensäule ausgegraben wird, wird ein Filterpapier auf das Lochsieb im Mikrokosmosgestell gelegt und mit Wasser angefeuchtet, damit beim Aufsetzen der Bodensäule das Filterpapier nicht beschädigt wird. Anschließend wird die Bodensäule vorsichtig ausgegraben und auf das Filterpapier gesetzt. Dann muß die Bodensäule fiir den Transport vom Feld ins Labor vorbereitet werden, wozu z.B. zusammengedrücktes Papier vorsichtig auf die Bodenoberfläche der Bodensäule gedrückt, der Transportdeckel aufgesetzt und mit Schrauben befestigt wird.

Im Falle einer gestörten Bodenprobe, wird zuerst im Feld die Lagerungsdichte des Bodens mit Hilfe kleiner Stichzylinder bestimmt. Dann wird der Boden vom landwirtschaftlichen Feld geholt und im Labor etwas vorgetrocknet, damit der Boden gesiebt werden kann. Je nach Bodentyp kann es nötig werden, daß der Boden nicht auf 2 mm (wie in den oben erwähnten Richtlinien der Bundesanstalt für Land- und Forstwirtschaft), sondern geeigneterweise auf 5 mm gesiebt wird, um eine bessere Bodenstruktur zu erhalten. Auch in diesem Fall wird vor Einfüllen des Bodens in den Zylinder ein Filterpapier auf das Lochsieb in der Mikrokosmoshalterung gelegt und mit Wasser angefeuchtet, bevor der Zylinder aufgesetzt und in der Mikrokosmoshalterung befestigt wird.

Nach dem Füllen des Zylinders mit einer ungestört entnommenen oder gestörten Bodenprobe sollte der Boden auf Feldkapazität bzw. maximale Wasserhaltekapazität eingestellt werden, um gleiche Verhältnisse in den Bodensäulen bei Versuchsbeginn zu gewährleisten. Zu diesem Zweck wird der komplette Mikrokosmos bei Raumtemperatur in ein Wasserbad mit einer Wasserhöhe von etwa 15 cm gestellt. Erst wenn alle Mikrokosmen eine nasse bzw. glänzende Bodenoberfläche zeigen, werden sie aus dem Wasserbad genommen und 24 Stunden stehen gelassen. Vorzugsweise wird, wie oben im Zusammenhang mit der Beregnungsflüssigkeit erwähnt, eine 0,01 molare Calciumchlorid-Lösung zum Einstellen der Feldkapazität verwendet. Anschließend wird das Unterdrucksystem aktiviert, vorzugsweise der Unterdruck in den Mikrokosmen auf maximal 200 mbar eingestellt, wodurch der Grundwasserstand unter Feldbedingungen simuliert wird.

Die vorgezogenen Pflanzen werden in einem geeigneten Stadium, z.B. Zweiblattstadium, eingesetzt, wobei zunächst die Pflanzenwurzeln unter fließendem Wasser gereinigt werden. Hierbei muß darauf geachtet werden, daß die Pflanzen nicht verletzt werden. Beim Einsetzen wird zunächst die oberste Bodenschicht entfernt und die Pflanzenwurzeln werden vorsichtig durch das Glasgefäß und die beiden Irisblenden geführt und gleichmäßig auf der Bodenoberfläche verteilt. Dann wird der vorher entfernte Boden wieder vorsichtig in den Mikrokosmos eingefüllt, leicht angedrückt und angegossen. Geeigneterweise läßt man die Pflanzen anschließend für ca. eine Woche anwachsen.

Die Bodenoberflächenapplikation der zu testenden Umweltchemikalie kann mit einer Hamiltonspritze, einer Airbrushpistole oder einer handelsüblichen landwirtschaftlichen Düse erfolgen. Dabei sollte das zu applizierende Volumen vorzugsweise die auf dem landwirdschaftlichen Feld angebrachte Menge an Spritzbrühe nicht überschreiten. Es ist darauf zu achten, daß das Pflanzenschutzmittel gleichmäßig über die Bodenoberfläche verteilt wird. Hierzu wird im Falle der Verwendung einer Hamiltonspritze diese geeigneterweise in immer kleiner werdenden Kreisen über die Bodenoberfläche geführt und das Pflanzenschutzmittel vorsichtig tröpfchenweise appliziert. Anschließend kann die Hamiltonspritze nochmals mit dem bereits vorher verwendeten Lösungsmittel (hierbei kann es sich auch um Wasser handeln) aufgezogen und der Spritzeninhalt nochmals gleichmäßig auf der Bodenoberfläche verteilt. Für die Simulation von Regenereignissen wird die Beregnungsvorrichtung je nach ausgewähltem Niederschlagsszenario entsprechend einem Zeitplan betätigt. Um die Bodenstruktur in den Bodensäulen nicht zu beeinträchtigen, wird, wie oben erwähnt, geeigneterweise mit einer 0,01 molaren CaCl₂-Lösung beregnet. Hierzu wird eine abgemessene Menge an Regenwasser zuerst in den Regenwasservorratsbehälter gegeben und der Mikrokosmos mit dem Regenwasserbehälter über einen Kunststoffschlauch verbunden. Vor dem Öffnen des Hahns des Regenwasservorratsbehälters wird vorzugsweise eine am Mikrokosmos angeordnete Entlüftungsschraube geöffnet, dann gewartet, bis der Kunststoffschlauch und der Deckel des Mikrokosmos entlüftet sind, und anschließend die Entlüftungsschraube am Mikrokosmos wieder geschlossen. Sollten nach der Beregnung keine Luftblasen im Mikrokosmosdeckel zu beobachten sein, kann bei der nächsten Bewässerung gegebenenfalls auf ein Entlüften des Deckels verzichtet werden. Als geeignetes Niederschlagsszenario kann beispielsweise dreimal pro Woche beregnet werden, wobei montags und mittwochs jeweils 80 ml und freitags 120 ml Regenwasser pro Mikrokosmos eingesetzt werden.

Für die Analyse des Verhaltens der zu untersuchenden Umweltchemikalie, insbesondere die Aufstellung einer Massenbilanz, können herkömmliche Methoden eingesetzt werden, wie sie beispielsweise bei Schroll R. und Scheunert I., supra, Schroll R. und Scheunert I. (1993) Chemosphere, Vol. 26, No. 9, Seiten 1631-1640, Schroll R. et al. (1994) Chemosphere, Vol. 28, No. 2, Seiten 297-303, und Gayler S. et al. (1995) Environ. Sci. & Pollut. Res., Vol. 2, No. 2, Seiten 98-103, beschrieben sind.

Für die Volatilitätsmessung werden die Polyurethan-Schäume aus den Gasfallen entfernt und mit Aceton oder anderen geeigneten Lösungsmitteln ausgewaschen, und die Lösungsmittelextrakte gegebenenfalls anschließend am Rotationsverdampfer eingeengt. Zur Szintillationsmessung wird ein geeigneter Szintillationscocktail, beispielsweise Ultima Gold XR (erhältlich von der Firma Canberra Packard, Frankfurt) verwendet.

Die in den Intensivflaschen enthaltene Mischung aus Ethanolamin und Diethylenglycolmonobutylether wird zu gewünschter Zeit in einen Meßzylinder abgelassen und das Volumen bestimmt. Aliquots werden in einem geeigneten Szintillationscocktail (z.B. Hionic Fluor von Canberra Packard, Frankfurt) zur Messung gebracht.

Während eines Versuchs, dessen Dauer möglichst mindestens ungefähr zwei Monate betragen sollte, werden sowohl die Volatilität und die ¹⁴CO₂-Entwicklung von der Bodenoberfläche als auch das Entweichen von ¹⁴C-organischen Verbindungen und ¹⁴CO₂ aus den Pflanzen gemessen. Die Probennahme erfolgt vorzugsweise nach einem strengen Zeitplan.

Volatile ¹⁴C-Bestandteile werden mittels der oben erwähnten Polyurethan-Fallen aufgefangen. Die Volatilität wird sowohl in den Mikrokosmen mit Pflanzen, als auch in den Mikrokosmen ohne Pflanzen bestimmt. Gleiches gilt für die ¹⁴CO₂-Entwicklung von der Bodenoberfläche, die ebenfalls in beiden Mikrokosmenarten gemessen wird.

Zur Bestimmung von aus den Pflanzen entweichenden organischen Verbindungen werden flüchtige ¹⁴C-Bestandteile mit Hilfe der PU-Fallen aufgefangen. Die Verflüchtigung wird mit dem Fallensystem an den Pflanzenkammern an den Mikrokosmen mit Pflanzen bestimmt. Gleiches gilt fiir die ¹⁴CO₂-Entwicklung aus den Testpflanzen.

Das Sickerwasser sollte mindestens wöchentlich untersucht werden, wozu für jeden Mikrokosmos zunächst das Volumen des Sickerwassers gemessen und anschließend jeweils ein Aliquot des Sickerwassers mit einem geeigneten Szintillationscocktail im Szintillationsmeßgerät zur Messung gebracht.

Für die qualitative Untersuchung des Sickerwassers nach Versuchsende wird das Sickerwasser über Festphasenextraktion extrahiert (z.B. Bond Elut der Firma Varian, oder z.B. LiChrolut EN der Firma Merck) und anschließend in einer HPLC qualitativ untersucht.

Nach Beendigung des Versuchs werden die Versuchspflanzen aufgearbeitet, wobei die Aufarbeitung vorzugsweise getrennt nach unter- und oberirdischen Pflanzenteilen erfolgt. Die Blätter und Stengel werden geeigneterweise in flüssigen Stickstoff getaucht und in einer Mühle pulverisiert. Das Pflanzenpulver kann anschließend mit entsprechenden Lösungsmitteln extrahiert und der Extrakt, gegebenenfalls nach Aufreinigung mittels Dünnschichtchromatographie, Gelpermeationschromatographie oder Gegenstromchromatographie, mittels einer HPLC qualitativ untersucht werden.

Zusätzlich werden die Pflanzenkammern innen mit Lösungsmittel, beispeilsweise Aceton oder Methanol, sorgfältig gewaschen, das gesamte Waschlösungsmittel gesammelt und am Rotationsverdampfer eingeengt. Nach Aufnahme in einer geeigneten Menge an Lösungsmittel wird im Szintillationszähler der Gehalt an radioaktiv markiertem Kohlenstoff bestimmt.

Schließlich werden auch die Bodenproben einer Rückstandsanalytik unterzogen, wobei die Bodenproben Schicht für Schicht extrahiert werden, die Menge an extrahierbarer ¹⁴C-Radioaktivität im Szintillationszähler erfaßt wird und mit Hilfe chromatographischer Methoden (HPLC, GC, MS) die einzelnen Substanzen identifiziert werden. Auf diese Weise ermöglicht die komplette Rückstandsanalytik die Aufstellung einer vollständigen Massenbilanz.

Im folgenden soll der Einsatz des erfindungsgemäßen Testsystems anhand von zwei Versuchsbeispielen erläutert werden.

### A) Analyse des Verhaltens des Herbizids Terbuthylazin

Das ¹⁴C-markierte Herbizid Terbuthylazin wurde in einem Mikrokosmenvesuch analysiert, wobei vier Mikrokosmen mit jeweils einer Maispflanze ausgestattet waren, während vier weitere Mikrokosmen keine Pflanzen aufwiesen.

Wie oben erwähnt, liegt ein entscheidender Vorteil der erfindungsgemäßen Anlage darin, daß sie u.a. die Bestimmung des Einflusses von Pflanzen auf den Abbau bzw. die Abbaurate von ¹⁴C-markierten Agrochemikalien ermöglicht. Da die Mikrokosmenanlage die direkte Messung sämtlicher Rückstände der von den Pflanzen über die Wurzeln aufgenommenen und über die Blätter in die Atmosphäre abgegebenen Chemikalien ermöglicht, kann durch Vergleich des Abbauverhaltens in "Mikrokosmen mit Pflanzen" mit dem in "Mikrokosmen ohne Pflanzen" der Einfluß der Pflanzen auf die Mineralisierung sichtbar gemacht werden.

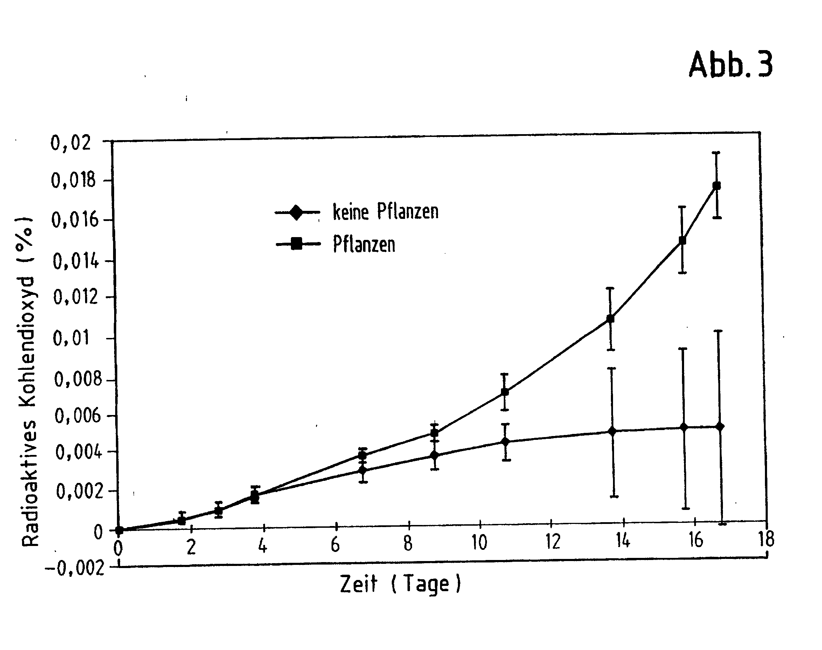

In Abbildung 3 ist die aufsummierte Menge an ¹⁴CO₂ in Prozent der ursprünglich applizierten Gesamtmenge gegen die Zeit für die verschiedenen Mikrokosmen ("mit Pflanzen" versus "ohne Pflanzen") dargestellt. Ab Versuchstag 11 ist ein signifikanter Unterschied in der Mineralisierung des Terbuthylazins erkennbar. In den Mikrokosmen mit Pflanzen wird das Herbizid stärker abgebaut als in den Mikrokosmen ohne Pflanzen. Somit konnte hier ein Einfluß von Maispflanzen auf die Mineralisierung von Terbuthylazin nachgewiesen werden.

### B) Analyse des Verhaltens des Herbizids Isoproturon

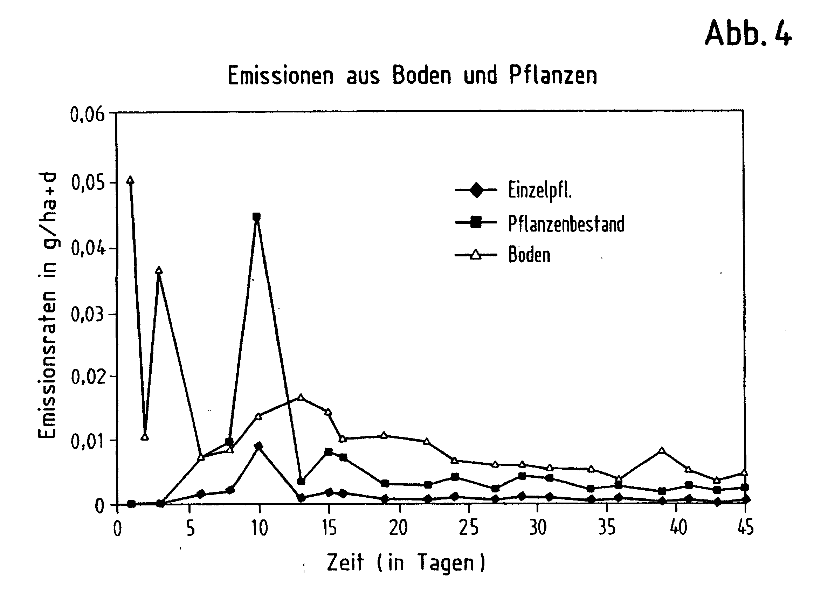

Abbildung 4 zeigt die Emmission des Herbizids Isoproturon von Boden- und Pflanzenoberflächen. Die Versuchsdaten sind in Form einer "Ratenkurve" (g/ha und Tag) dargestellt. Das Herbizid wurde zu Versuchsbeginn auf die Bodenoberfläche appliziert. Die Verflüchtigungsraten von der Bodenoberfläche sind in der Kurve "Boden" zu erkennen, wobei die Kurve den Mittelwert aus vier Versuchen darstellt.

Von den in diesen Versuchen eingesetzten Weizenpflanzen (je eine Pflanze pro Mikrokosmos, insgesmamt vier Mikrokosmen) wurden die Herbizidrückstände über die Pflanzenwurzeln aufgenommen, anschließend in die Blätter transloziert und über die Blätter in die Atmosphäre abgegeben (Kurve "Einzelpflanzen"). Da die Pflanzdichte pro Mikrokosmos jedoch nicht der im Freilandversuch entspricht, wurde dieser Wert auf die tatsächlich auf dem landwirtschaftlichen Feld vorhandene Pflanzendichte hochgerechnet (Kurve "Pflanzenbestand"). Man erkennt, daß über einen längeren Zeitraum betrachtet, die "Emission" von Pflanzenschutzmittelrückständen aus Pflanzen der Verflüchtigung von Pflanzenschutzmitteln von Bodenoberflächen sehr nahe kommen kann.

## Patentansprüche

1. Labortestsystem zur Untersuchung des Verhaltens von Umweltchemikalien, insbesondere Pflanzenschutzmitteln, umfassend mindestens ein Behältnis, welches mit einer gestörten oder ungestörten Bodensäule gefüllt ist, eine oberhalb der Bodenoberfläche angeordnete Beregnungsvorrichtung, eine Ventilatorvorrichtung, die eine gleichmäßige Windbewegung oberhalb der Bodenoberfläche erzeugt, und ein Unterdrucksystem zur Einstellung des Grundwasserspiegels innerhalb der Bodensäule.

2. Labortestsystem nach Anspruch 1, umfassend mindestens 4 mit einer gestörten oder ungestörten Bodensäule gefüllte Behältnisse.

3. Labortestsystem nach Anspruch 1 oder 2, das mindestens eine mit einer Kühlzone ausgestattete Intensivwaschflasche zur Auswaschung der durch das System geleiteten Luft umfaßt.

4. Verfahren zur Untersuchung des Verhaltens von Umweltchemikalien, insbesondere Pflanzenschutzmitteln, bei dem ein Labortestsystem nach einem der vorangehenden Ansprüche eingesetzt wird.

5. Verfahren nach Anspruch 4, bei dem die Umweltchemikalien radioaktiv markiert, insbesondere ¹⁴C-markiert, sind.

6. Verfahren nach Anspruch 4 oder 5, bei dem das Verhalten von Umweltchemikalien vergleichsweise in Anwesenheit und in Abwesenheit von Pflanzen untersucht wird.

## Claims

1. A laboratory test system for testing the behavior of environmental chemicals, in particular plant protection agents, comprising at least one container filled with a disturbed or undisturbed soil column, an irrigation device arranged above the soil surface, a ventilator device that generates a uniform wind movement, and a vacuum system for adjusting the subsoil water level within said soil column.

2. The laboratory test system according to claim 1, comprising at least 4 containers filled with a disturbed or undisturbed soil column.

3. The laboratory test system according to claim 1 or 2, comprising at least one intensive purifying jar equipped with a cooling zone for purifying the air guided through said system.

4. A method of testing the behavior of environmental chemicals, in particular plant protection agents, wherein a laboratory test system according to any one of the preceding claims is used.

5. The method according to claim 4, wherein the environmental chemicals are radioactively labeled, in particularly ¹⁴C-labeled.

6. The method according to claim 4 or 5, wherein the behavior of environmental chemicals is tested comparatively in the presence or in the absence of plants.

## Revendications

1. Système de test en laboratoire pour l'examen du comportement de produits chimiques environnementaux, en particulier de moyens de protection des plantes, comportant au moins un récipient empli d'une carotte de prélèvement de sol perturbée ou non perturbée, un dispositif d'arrosage disposé au-dessus de la surface du sol, un dispositif de ventilation créant un vent régulier au-dessus de la surface du sol, et un système de dépression pour le réglage du niveau de la nappe d'eau souterraine dans la carotte de prélèvement de sol.

2. Système de test en laboratoire selon la revendication 1, comprenant au moins 4 récipients emplis d'une carotte de prélèvement de sol perturbée ou non perturbée.

3. Système de test en laboratoire selon la revendication 1 ou 2, comprenant au moins un flacon de lavage poussé comportant une zone de refroidissement pour le lavage de l'air traversant le système.

4. Procédé d'examen du comportement de produits chimiques environnementaux, en particulier de moyens de protection des plantes, dans lequel un système de test en laboratoire selon l'une des revendications précédentes est mis en oeuvre.

5. Procédé selon la revendication 4 dans lequel les produits chimiques environnementaux sont marqués de manière radioactive, en particulier par du ¹⁴C.

6. Procédé selon l'une des revendications 4 ou 5 dans lequel le comportement de produits chimiques environnementaux est examiné de manière comparative en présence et en l'absence de plantes.
